# EUROPEAN PATENT SPECIFICATION

(11) **EP 2 705 839 B1**
(45) Date of publication and mention of the grant of the patent: **21.02.2018**
(21) Application number: 13183603.3
(22) Date of filing: 09.09.2013
(51) Int. Cl.: A61K 9/20, A61K 31/44

(54) **PHARMACEUTICAL COMPOSITION COMPRISING LACIDIPINE AND PROCESS OF PREPARATION**
PHARMAZEUTISCHE ZUSAMMENSETZUNG MIT LACIDIPIN UND HERSTELLUNGSVERFAHREN
COMPOSITION PHARMACEUTIQUE CONTENANT DE LA LACIDIPINE ET PROCÉDÉ DE PRÉPARATION

(30) Priority: 10.09.2012 EP 12183762
(43) Date of publication of application: 12.03.2014
(73) Proprietor: Rivopharm SA, 6928 Manno (CH)
(72) Inventor: Poli, Piero, CH-6900 Lugano (CH); Carcano, Michela, I-22026 Maslianico, COMO (IT); Vecchi, Gabriele, I-22100 Como (IT)
(74) Representative: Croce, Valeria

(56) References cited:
- WO-A1-2006/113309
- KR-A- 20110 032 608
- US-A- 6 071 939

## Description

### Technical field

The instant invention relates to a pharmaceutical composition comprising lacidipine and a process for preparing said composition.

### Background of the invention

Lacidipine (3,5-diethyl 4- {2-[(1*E*)-3-(*tert*-butoxy)-3-oxoprop-1-en-1-yl]phenyl}-2,6-dimethyl-1,4-dihydropyridine-3,5-dicarboxylate) is a dihydropyridine calcium channel antagonist. Lacidipine is mainly used for the prevention and the treatment of hypertension. The chemical formula of lacidipine is as follows:

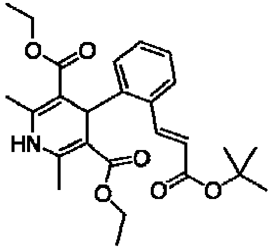

Its synthesis is described in U.S. patent 4,801,599.

Lacidipine was primarily marketed under trade name Motens® or Lacipil®, as film-coated tablets which comprise lactose and polyvinylpyrrolidone as main excipients.

Since lacidipine is poorly soluble in water, providing pharmaceutical compositions for the oral delivery of lacipidine has been a real challenge for a long time.

US 4,801,599 describes the preparation of lacidipine tablets by both wet granulation and direct compression.

International patent application WO 2006/113309 suggests reducing the size of lacidipine particles in order to improve its bioavailability.

Finally, International application WO 95/08987 describes a method for preparing a solid pharmaceutical composition comprising a dihydropyridine derivatives, said method comprising the steps of preparing a solution containing the said dihydropyridine derivative and a surfactant, and spraying the said solution onto a carrier under a stream of heated air. KR 2011/0032608 discloses a solid pharmaceutical composition comprising lacidipine and polyvinylpyrrolidone, wherein the weight ratio of lacidipine to polyvinylpyrrolidone is about 10/90. The composition is obtained by dissolving PVP in acetone/ethanol followed by solubilization of lacidipine. Lactose granules were then added followed by solvent evaporation (wet granulation). Mg stearate was then added and the mixture was then compressed into tablets. The final tablets comprise 4mg lacidipine/tablet. The tablets are useful in the treatment of hypertension. There is still a need for alternate lacidipine pharmaceutical compositions and methods for preparing them.

### Summary of the invention

The invention relates to a process for preparing a solid pharmaceutical lacidipine composition comprising the steps of:
a) providing a solution of polyvinylpyrrolidone and lacidipine in a polar solvent wherein the weight ratio of lacidipine to polyvinylpyrrolidone is about 10/90,
b) spraying and drying in a fluid bed the solution of step a) onto a solid diluent, so as to obtain granules,
c) optionally, adding to the granules obtained in step b) at least one additional excipient preferably selected from a glidant, a lubricant, a diluent and combinations thereof, and
d) shaping the resulting granules into a solid form, preferably into a tablet.

In some embodiments, the process according to the invention is characterized by one or several of the following features:
- the polyvinylpyrrolidone of step a) has an average molecular weight ranging from 10 000 to 1 000 000 g.mol⁻¹, preferably from 20 000 to 60 000 g.mol⁻¹
- the polar solvent provided in step a) is selected from ethanol and ethanol/water mixtures comprising at least 80% (v/v) of ethanol,
- the diluent in step b) is selected from the group consisting of microcrystalline cellulose, mono-saccharides, preferably lactose, di-saccharides and combinations thereof,
- step b) is performed in a fluidized bed processor,
- the polar solvent is evaporated during step b) by the mean of a stream of gas,
- in step c), a diluent, preferably lactose, and a lubricant, preferably magnesium stearate, are added to the granule,
- the weight ratio between the diluent used in step b) and the diluent added in step c) ranges from 2 to 3, preferably from 2.1 to 2.5, and
- the process further comprises coating the solid form obtained in step d).

### Detailed description of the invention

### - General description of the process according to the invention

Surprisingly, the Applicant showed that the weight ratio of lacidipine to polyvinylpyrrolidone (also called hereunder PVP or povidone) has a dramatic impact on the pharmacokinetic parameters of lacidipine *in vivo* upon oral administration. Indeed, a decrease of lacidipine/PVP ratio (e.g. from 1/9 to 1/10) significantly increased the Cₘₐₓ value *in vivo*, which may lead to an increase of side-effects in patients, especially patients under chronic administration.

Such an impact on Cₘₐₓ could not have been anticipated from *in vitro* data since the Applicant showed that tablets having distinct lacidipine/PVP weight ratio (e.g. from 1/10 to 1/9) actually display the same *in vitro* dissolution profile for lacidipine.

Controlling Cₘₐₓ value without impairing the overall bioavailability of lacidipine is indeed a real challenge for generic companies. According to the bioequivalence guidelines from the European Medecines Agency (CHMP, guideline on the investigation of bioequivalence, January 20, 2010), the parameters to analyse in order to determine whether a "drug" is bioequivalent to the reference drug and qualify as a generic drug are AUC and Cₘₐₓ (upon a single dose administration under fasting conditions). For these parameters, the 90% confidence interval for the ratios of the generic drug and the reference drug should be contained within the acceptance interval of 80.00%-125.00%. In the case of lacidipine formulations, the Applicant showed that such criteria can be met, in respect to Lacipil® as reference drug, by precisely controlling the weight ratio of lacipidine to binding agent, in particular the ratio of lacidipine to PVP.

As used herein, a weight ratio of about 1/9 means a weight ratio of 0.111 ± 0.005.

As used herein, a PVP (also called povidone or polyvinylpyrrolidone) refers to a polymer made from the monomer N-vinylpyrrolidone.

In some embodiments, the polyvinylpyrrolidone is selected from PVP having an average molecular weight from 10 000 to 1 000 000 g.mol⁻¹ (which means that the average weight of the polymers present in said PVP ranges from 10 000 to 1 000 000 g.mol⁻¹). An average weight from 1.10⁴ to 100.10⁴ g.mol⁻¹ encompasses an average weight from 1.10⁴ to 10.10⁴, from 10.10⁴ to 20.10⁴, from 20.10⁴ to 30.10⁴, from 30.10⁴ to 40.10⁴, from 40.10⁴ to 50.10⁴, and from 50.10⁴ to 100.10⁴ g.mol⁻¹. In some embodiments, the polyvinylpyrrolidone has an average molecular weight from about 20 000 to 80 000 g.mol⁻¹, preferably from about 20 000 to 60 000 g.mol⁻¹ such that from about 30 000 g.mol⁻¹ to about 50 000 g.mol⁻¹

Another way to define appropriate polyvinylpyrrolidones is the K-value. The K-values are derived from the relative viscosity in water and may be calculated using Fikentscher's equation. The K-value is generally used in order to express the average molecular weight of povidone and generally forms a part of the commercial name of povidones. The correlation between K-value and average molecular weight of povidone is for example shown in Handbook of Pharmaceuticals Excipients, American Pharmaceutical Association (Pharmaceutical Press; 6th revised edition, 2009).

Typically, appropriate PVPs have a K-value from 17 to 90, preferably from 17 to 60, more preferably from 25 to 35. For example, PVP may have a K-value from 25 to 30. Lacidipine present within the composition may be crystalline, amorphous or a mixture of amorphous and crystalline forms. In a preferred embodiment, the lacidipine present in the composition of the invention is in an essentially amorphous form.

As used herein, "lacidipine in an essentially amorphous form or amorphous state" means that at least 98%, preferably at least 99% of the lacidipine present in the pharmaceutical composition is amorphous. Preferably, lacidipine present in the pharmaceutical composition is amorphous.

Without being bound by any theory, the Applicant believes that the formulation process may have some impacts on the bioavailability of lacidipine. Preferred processes are those which allow an intimate mixture of PVP with lacidipine.

Without being bound by any theory, the Applicant believes that such a step promotes the formation of a solid dispersion in which lacipidine in an essentially amorphous state is dispersed within PVP matrix on the surface of diluent while enabling granulation.

Accordingly, in a further aspect, the invention relates to a process for preparing a solid pharmaceutical lacidipine composition, the said process comprises the steps of:
a) providing a solution of polyvinylpyrrolidone and lacidipine in a polar solvent wherein the weight ratio of lacidipine to polyvinylpyrrolidone is about 10/90,
b) spraying and drying in a fluid bed the solution of step a) onto a solid diluent, so as to obtain granules,
c) optionally, adding to the granules obtained in step b) at least one additional excipient preferably selected from a glidant, a lubricant, a diluent and combinations thereof, and
d) shaping the resulting granules into a solid form, preferably into a tablet.

As used herein, "a solid form" means that one or several identical forms may be prepared by the process of the invention. As used herein, a solid form encompasses all the pharmaceutical forms comprising lacidipine in solid state such as pills, tablets, hard gelatin capsules and the like. Preferably, the solid form refers to an uncoated tablet or a film-coated tablet.

In step a), the solvent is selected so as to promote the dissolution of PVP and lacidipine. The solvent is preferably selected among solvents recommended by the European Pharmacopeia. In a preferred embodiment, the solvent is selected from ethanol and ethanol/water mixtures which comprise at least 80%, preferably at least 90%, more preferably at least 95% of ethanol, the percentage being expressed by volume as compared to the total volume of the mixture. In some embodiments, the solution provided in step a) is a clear solution, which means that PVP and lacidipine are completely dissolved.

In step b), the solid diluent may be virtually any diluent commonly used in pharmaceutical formulations. In particular, the diluent may be selected from any pharmaceutically acceptable agents or combinations of agents that increase the bulk volume of the active ingredient so that production of a tablet of practical size is possible. In some embodiments, the diluent is in the form of a powder. In some further embodiments, the diluent is selected from the group consisting of microcrystalline cellulose, mono-saccharides, di-saccharides and hydrates thereof. For example, monosaccharides encompass, without being limited to, lactose such as anhydrous lactose and lactose monohydrate, mannitol, glucose and sorbitol.

An example of disaccharide is sucrose. In some embodiments, the diluent is selected among the group consisting of lactose, mannitol, microcrystalline cellulose and mixture thereof.

Preferably, the diluent used in step b), and eventually in subsequent steps, is lactose such as lactose monohydrate.

The solvent of the solution is at least partially evaporated during step b). The evaporation of the solvent may result from the spraying/drying process. In some embodiments, the evaporation of the solvent may be promoted by the mean of a stream of gas, typically by the mean of a stream of heated air.

In some embodiments, step b) and/or step c) may be followed by a step of drying the granules. This drying step may be performed by subjecting the granules to a stream of gas or by heating the granules at a temperature from 30°C to 80°C.

In advantageous embodiments, step b) is performed in a fluidized bed processor. In such a case, the diluent, for e.g. lactose, may be suspended in the heated air of the fluid bed. The solution containing PVP and lacipidine may be sprayed on fluidized diluent by the mean of nozzles, which promotes the formation of granules. The granules may be then dried with heated air. As an alternative, the drying and the spraying may occur simultaneously.

As an example, one may use commercially available fluid-bed apparatus equipped with an insert for top spray (droplets are sprayed from the top) or bottom spray using a Wurster-type column, or tangential spray using a rotor disk (droplets are sprayed from a side of the bottom). In a Wurster fluid-bed apparatus, diluent particles are entrained in a high velocity gas and pass through the Wurster column where fine droplets of solution comprising lacidipine and PVP are sprayed from a jet nozzle. The gas stream is heated to allow the evaporation of the solvent from the sprayed solution. The result is to deposit a plurality of particles of lacidipine together with PVP onto diluent substrate.

Step c) is optional. However, it may be advantageous to mix the granules with at least one excipient. Such a step may enable to improve the pharmaco-technical properties and/or the pharmacokinetics properties of the final composition.

In some embodiments step c) comprises mixing the granules obtained in step b) with at least one lubricant and at least one diluent. The diluent used in step c) may be identical or distinct from that used in step b). For instance, the diluent in step c) may be selected among the group consisting of lactose, mannitol, microcrystalline cellulose and mixture thereof. Preferably, the diluent used in step c) is spray-dried. In some embodiments, the diluent added in step c) is lactose such as lactose monohydrate. For example, an appropriate diluent is a lactose monohydrate which has been spray-dried.

In some embodiments, the diluent used in step b) is lactose and the diluent used in step c) is lactose, mannitol or microcrystalline cellulose.

In other embodiments, the diluent used in step b) is mannitol and the diluent used in step c) is lactose, mannitol or crystalline cellulose.

In some further embodiments, the diluent used in step b) is microcrystalline cellulose and the diluent used in step c) is lactose, mannitol or crystalline cellulose.

In some other embodiments, the diluents used in both steps b) and c) are lactose.

Without being bound by any theory, the applicant believes the ratio between intragranular and extragranular amounts of diluent may affect the workabibility of the product during the manufacturing steps as well as the characteristics of the final composition and consequently the lacidipine release in vivo. Accordingly, in some preferred embodiments, the weight ratio of the diluent used in step b) to the diluent added in step c) ranges from 2 to 3, preferably from 2.1 to 2.5. Typically, said ratio may be about 2.3.

The lubricant added in step c) may be any stearate salt such as magnesium stearate, calcium stearate or zinc stearate. Magnesium stearate is preferred.

Step d) may consist in compacting granulates so as to obtain a solid form such as a tablet. This step may be performed by conventional means, for example, by using a tablet press.

The process according to the invention may be performed by using excipients and device which are commercially available.

The process according to the invention may comprise one or several additional steps. For example, the excipients and the active ingredient may be sieved before use and/or the granules may be sieved or sized before being put into shape. The process may also comprise one or several drying steps.

In some preferred embodiments, the process of the invention further comprises a step of coating the solid form. Such a coating may be performed by standard procedures using conventional coating agent and a coating pan.

The coating step may thus comprise the sub-steps of:
- spraying the coating agent onto the solid form, and
- drying the coated solid form.

The coating agent is preferably put into a solvent, such as water, ethanol or diethyl ether, before being spraying onto the solid form. The coating agent may comprise a film-forming agent. Examples of a film-forming agent are a water-soluble cellulose derivative such as methylcellulose, hydroxypropylmethylcellulose, hydroxy-propylcellulose, hydroxyethylcellulose and sodium carboxymethylcellulose, a water-insoluble cellulose derivative such as ethylcellulose, cellulose acetate phthalate and hydroxypropylmethylcellulose phthalate, polyvinylpyrrolidone, polyvinyl acetate phthalate, polyvinyl acetate, polyvinyl alcohols, polyethylene glycol, fats and sugar syrup. In some embodiments, the film forming agent is selected from polymers soluble in water. The coating agent is generally a mixture comprising at least one film-forming agent together with one or several excipients such as a binder and/or a pigment (for example iron oxide, aluminum oxide and/or titanium dioxide). Appropriate coating agents are Opadry® provided by Colorcon, based on HPMC (hydroxypropylmethylcellulose) or based on PVA (polyvinyl alcohol).

### - Particular embodiments of the process

In a particular embodiment, the process for preparing a solid pharmaceutical composition of lacidipine comprises the steps of:
a) providing a solution of polyvinylpyrrolidone and lacidipine in ethanol or in ethanol/water mixtures having at least 80% of ethanol per volume, wherein the weight ratio of lacidipine to polyvinylpyrrolidone is about 10/90,
b) spraying and drying in a fluid bed the solution of step a) onto a solid diluent, preferably lactose monohydrate, so as to obtain granules, the said step b) being performed in a fluidized bed processor,
c) adding to the granules obtained in step b) at least one lubricant, preferably magnesium stearate, and at least one diluent, preferably lactose monohydrate, so that the weight ratio of the diluent used in step b) to the diluent used in step c) ranges from 2 to 3, preferably 2.1 to 2.6,
d) shaping the resulting granules into a solid form, preferably into a tablet, and
e) coating the tablet of step d).

Further aspects and advantages of the present invention will be disclosed in the following examples, which should be considered as illustrative and not limiting the scope of the present application.

### EXAMPLE 1: Preparation of pharmaceutical tablets according to the invention

Various film-coated tablets were prepared as follows:
1. Sieve the diluent and transfer it into the fluid bed,
2. In a suitable container dissolve povidone (PVP) in the solvent,
3. Add lacidipine to the povidone solution and stir up to reach a complete solubilisation
4. Evaporate the solvent by spraying the solution on the diluent into the fluid bed so to obtain a granulate. The temperature of the product may be about 30°C-40°C,
5. Add the remaining excipients to the granulate of step 4)
6. Tablet the final blend,
7. Coated the final tablets with Opadry coating agent in water.

The Tables 1 hereunder show the amounts of excipients used for preparing the tablets according to the inventive as well as comparative tablets. Each tablet of the invention differs from the corresponding comparative tablet by the weight ratio of lacidipine to povidone.

During the process, ethanol and water were removed. Each final tablet contains 6 mg of lacidipine.

| | **Tablet n°1 (Comparative) Weight ratio lacidipine/PVP : 1/10** | **Tablet n°2 (Inventive) Weight ratio lacidipine/PVP : 1/9** |
|---|---|---|
| **Granulate** | | |
| Lacidipine | 6,00 | 6,00 |
| Povidone | 60,00 | 54,00 |
| Lactose monohydrate | 267,00 | 273,00 |
| Ethanol* | 142,50 | 126,00 |

| **Final blend (extragranular excipients)** | | |
|---|---|---|
| Lactose monohydrate (Lactose spray dried) | 114,50 | 114,50 |
| Magnesium stearate | 2,50 | 2,50 |

| **Coating agent** | | |
|---|---|---|
| Opadry based on hypromellose | 11,30 | 11,30 |
| Purified water* | 83,00 | 83,00 |
| ***Total*** | ***461,30 mg*** | ***461,30 mg*** |

| | **Tablet n°3 (Comparative) Weight ratio lacidipine/PVP : 1/10** | **Tablet n°4 (Inventive) Weight ratio lacidipine/PVP : 1/9** |
|---|---|---|
| **Granulate** | | |
| Lacidipine | 6,00 | 6,00 |
| Povidone | 60,00 | 54,00 |
| Lactose monohydrate | 200,00 | 206,00 |
| Ethanol* | 142,50 | 126,00 |
| Purified water* | 17,50 | 14,00 |

| **Final blend (extragranular excipients)** | | |
|---|---|---|
| Lactose monohydrate (Lactose spray dried) | 181,50 | 181,50 |
| Magnesium stearate | 2,50 | 2,50 |

| **Coating agent** | | |
|---|---|---|
| Opadry based on hypromellose | 11,30 | 11,30 |
| Purified water* | 83,00 | 83,00 |
| ***Total*** | ***461,30 mg*** | ***461,30 mg*** |

| | **Tablet n°5 (Comparative) Weight ratio lacidipine/PVP : 1/10** | **Tablet n°6 (Inventive) Weight ratio lacidipine/PVP : 1/9** |
|---|---|---|
| **Granulate** | | |
| Lacidipine | 6,00 | 6,00 |
| Povidone | 60,00 | 54,00 |
| Microcrystalline cellulose (Avicel PH 101) | 200,00 | 206,00 |
| Ethanol* | 133,50 | 122,00 |

| **Final blend (extragranular excipients)** | | |
|---|---|---|
| Lactose monohydrate | 181,50 | 181,50 |
| (Lactose spray dried) | | |
| Magnesium stearate | 2,50 | 2,50 |

| **Coating agent** | | |
|---|---|---|
| Opadry based on hypromellose | 11,30 | 11,30 |
| Purified water* | 83,00 | 83,00 |
| ***Total*** | ***461,30 mg*** | ***461,30 mg*** |

| | **Tablet n°7 (Comparative) Weight ratio lacidipine/PVP : 1/10** | **Tablet n°8 (Inventive) Weight ratio lacidipine/PVP : 1/9** |
|---|---|---|
| **Granulate** | | |
| Lacidipine | 6,00 | 6,00 |
| Povidone | 60,00 | 54,00 |
| Mannitol (Pearlitol 160) | 250,00 | 256,00 |
| Ethanol* | 142,50 | 126,00 |

| **Final blend (extragranular excipients)** | | |
|---|---|---|
| Mannitol spray dried (Pearlitol 200SD) | 129,50 | 129,50 |
| Magnesium stearate | 4,50 | 4,50 |

| **Coating agent** | | |
|---|---|---|
| Opadry based on hypromellose | 11,30 | 11,30 |
| Purified water* | 83,00 | 83,00 |
| ***Total*** | ***461,30 mg*** | ***461,30 mg*** |

| | | |
|---|---|---|
| *Ethanol and water are removed during the process | | |

| | **Tablet n°9 (Comparative) Weight ratio lacidipine/PVP : 1/10** | **Tablet n°10 (Inventive) Weight ratio lacidipine/PVP : 1/9** |
|---|---|---|
| **Granulate** | | |
| Lacidipine | 6,00 | 6,00 |
| Povidone | 60,00 | 54,00 |
| Lactose milled (Granulac 200) | 267,00 | 273,00 |
| Ethanol* | 142,50 | 126,00 |

| **Final blend (extragranular excipients)** | | |
|---|---|---|
| Microcrystalline cellulose (Avicel PH 102) | 114,50 | 114,50 |
| Magnesium stearate | 2,50 | 2,50 |

| **Coating agent** | | |
|---|---|---|
| Opadry based on hypromellose | 11,30 | 11,30 |
| Purified water* | 83,00 | 83,00 |
| ***Total*** | ***461,30 mg*** | ***461,30 mg*** |

| | **Tablet n°11 (Comparative) Weight ratio lacidipine/PVP : 1/10** | **Tablet n°12 (Inventive) Weight ratio lacidipine/PVP : 1/9** |
|---|---|---|
| **Granulate** | | |
| Lacidipine | 6,00 | 6,00 |
| Povidone | 60,00 | 54,00 |
| Lactose milled | 285,00 | 291,00 |
| Ethanol* | 142,50 | 126,00 |
| Purified water* | 17,50 | 14,00 |

| **Final blend (extragranular excipients)** | | |
|---|---|---|
| Mannitol spray dried (Pearlitol 200SD) | 94,50 | 94,50 |
| Magnesium stearate | 4,50 | 4,50 |

| **Coating agent** | | |
|---|---|---|
| Opadry based on hypromellose | 11,30 | 11,30 |
| Purified water* | 83,00 | 83,00 |
| ***Total*** | ***461,30 mg*** | ***461,30 mg*** |

### EXAMPLE 2: In vitro dissolution profile of lacidipine for tablet n°1 (comparative) and tablet n°2 (inventive) as compared to drug reference (lacipil® 6 mg).

In vitro dissolution assays were performed using the following parameters:
- Medium: 600 ml of buffer at pH 6,8 + 0,4675% w/v of Tween 20
- Apparatus 2: Paddle at 50 RPM
- Temperature: 37°C

The results are shown in table 2 hereunder. It clearly appears that the three tablets provide similar dissolution profile of lacidipine *in vitro*.

**Table 2: in vitro dissolution kinetic of lacidipine for tablet n°1 (comparative), tablet n°2 (inventive) and reference tablet (Lacipil® - 6 mg)**

| | Tablet n°1 (comparative) | Tablet n°2 (inventive) | Lacipil® tablet (6mg) |
|---|---|---|---|
| **TIME (min)** | % of dissolved lacidipine | % of dissolved lacidipine | % of dissolved lacidipine |
| 0 | 0.0 | 0.0 | 0.0 |
| 10 | 36.5 | 34.7 | 34.9 |
| 15 | 56.0 | 56.1 | 57.4 |
| 20 | 72.4 | 73.9 | 77.9 |
| 30 | 85.5 | 89.4 | 94.2 |
| 45 | 93.8 | 92.7 | 95.3 |

### EXAMPLE 3: Assessments of pharmacokinetic parameters of tablet n°1 (comparative) and tablet n°2 (inventive as compared to drug reference (Lacipil® 6 mg)

A single center, randomized, single dose, laboratory-blinded, comparative bioavailability study was conducted under fasting conditions on both test formulations (tablet n°1 and tablet n°2) against the reference product Lacipil® 6mg. The rate and extent of absorption of lacidipine were measured and compared following a single dose (1 x 6 mg) of film-coated tablet. The pharmacokinetic results are summarized in tables 3 and 4 below:

**Table 3: Pharmacokinetic parameters for tablet n°1 (comparative) as compared to drug reference.**

| **PARAMETER** | **GEOMETRIC LSMEANS *** | | **RATIO (%)** | **90% CONFIDENCE LIMITS (%)** | |
|---|---|---|---|---|---|
| | **Tablet n°1** | **REFERENCE** | | **LOWER** | **UPPER** |
| Cₘₐₓ | 5061.9 | 4222.3 | 119.88 | 106.68 | **134.73** |
| AUC_{T} | 15253.8 | 13954.1 | 109.31 | 99.09 | 120.59 |

| | | | | | |
|---|---|---|---|---|---|
| * units are pg/mL for Cₘₐₓ and pg·h/mL for AUC_{T} | | | | | |

For Cₘₐₓ, the upper limit for the 90% confidence interval was outside the acceptance range of 80.00% to 125.00%. In other words, a lacipidipine/PVP weight ratio of 1/10 greatly increased Cmax value as compared to drug reference. The bioequivalence criteria were not met for tablet n°1 whereas tablet n°1 displayed a similar *in vitro* dissolution profil to that of reference drug.

**Table 4: Pharmacokinetic parameters for tablet n°2 (inventive) as compared to drug reference.**

| **PARAMETER** | **GEOMETRIC LSMEANS*** | | **RATIO (%)** | **90% CONFIDENCE LIMITS (%)** | |
|---|---|---|---|---|---|
| | **Tablet n°2** | **REFERENCE** | | **LOWER** | **UPPER** |
| Cₘₐₓ (ng/mL) | 8.669 | 8.389 | 103.3 | 91.87 | 116.25 |
| AUC_{T} (ng/mL) | 31.965 | 32.210 | 99.2 | 91.08 | 108.12 |

It appears that tablet n°2 met the bioequivalence criteria with respect to the rate and extent of absorption of Lacidipine. Such results illustrate the great impact of lacipidine/PVP weight ratio on lacipidine pharmacokinetics *in vivo*, which cannot be anticipated from *in vitro* dissolution profiles.

Also tablets No. 4, 6, 8, 10 and 12 show pharmacokinetic parameters inside the confidence limits of 80-125%.

## Claims

1. A process for preparing a solid pharmaceutical lacidipine composition comprising the following steps:
a) providing a solution of polyvinylpyrrolidone and lacidipine in a polar solvent wherein the weight ratio of lacidipine to polyvinylpyrrolidone is about 10/90,
b) spraying and drying in a fluid bed the solution of step a) onto a solid diluent, so as to obtain granules
c) optionally, adding to the granules obtained in step b) at least one additional excipient preferably selected from a glidant, a lubricant, a diluent and combinations thereof, and
d) shaping the resulting granules into a solid form, preferably into a tablet.

2. The process according to claim 1 wherein the polyvinylpyrrolidone of step a) has an average molecular weight from 10 000 to 1 000 000 g.mol⁻¹, preferably from 20 000 to 60 000 g.mol⁻¹.

3. The process of claims 1 or 2 wherein the polar solvent provided in step a) is selected from ethanol and ethanol/water mixtures comprising at least 80% (v/v) of ethanol.

4. The process according to anyone of claims 1 to 3 wherein the diluent in step b) is selected from the group consisting of microcrystalline cellulose, mono-saccharides, preferably lactose, di-saccharides and combinations thereof.

5. The process according to anyone one of claims 1 to 4 wherein step b) is performed in a fluidized bed processor.

6. The process according to anyone of claims 1 to 5 wherein in step c), a diluent, preferably lactose, and a lubricant, preferably magnesium stearate, are added to the granules.

7. The process according to claim 6 wherein the weight ratio between the diluent used in step b) and the diluent added in step c) ranges from 2 to 3, preferably from 2.1 to 2.5.

8. The process according to anyone of claims 1 to 7 further including the step of coating the solid form obtained in step d).

9. The process according to any one of claims 1 to 8, further including, after step b) and/or step c), a step of drying the granules by subjecting the granules to a stream of gas or by heating the granules at a temperature from 30°C to 80°C.

10. The process according to any one of claims 1 to 9, wherein in step b) the evaporation of the solvent is promoted by means of a stream of gas.

11. The process according to any one of claims 1 to 10, wherein the excipients and the active ingredient are sieved before use and/or the granules are sieved or sized before being put into shape.

## Patentansprüche

1. Verfahren zur Herstellung einer festen pharmazeutischen Lacidipin-Zusammensetzung umfassend die folgenden Schritte:
a) Bereitstellen einer Lösung von Polyvinylpyrrolidon und Lacidipin in einem polaren Lösungsmittel, wobei das Gewichtsverhältnis von Lacidipin zu Polyvinylpyrrolidon ungefähr 10/90 beträgt,
b) Sprühen und Trocknen der Lösung aus Schritt a) in einem Fließbett auf ein festes Verdünnungsmittel, so dass Granulate erhalten werden,
c) optional, Zugeben mindestens eines zusätzlichen Hilfsstoffes zu den in Schritt b) erhaltenen Granulaten, bevorzugt ausgewählt aus einem Gleitmittel, einem Schmiermittel, einem Verdünnungsmittel und Kombinationen hiervon, und
d) Formen der resultierenden Granulate in eine feste Form, vorzugsweise in eine Tablette.

2. Verfahren nach Anspruch 1, worin das Polyvinylpyrrolidon von Schritt a) ein durchschnittliches Molekulargewicht von 10.000 bis 1.000.000 g·mol⁻¹, bevorzugt 20.000 bis 600.000 g·mol⁻¹ aufweist.

3. Verfahren nach Anspruch 1 oder 2, worin das in Schritt a) bereitgestellte polare Lösungsmittel ausgewählt ist aus Ethanol und Ethanol/Wasser-Mischungen umfassend mindestens 80% (v/v) Ethanol.

4. Verfahren nach einem der Ansprüche 1 bis 3, worin das Verdünnungsmittel in Schritt b) ausgewählt ist aus der Gruppe bestehend aus mikrokristalliner Zellulose, Monosacchariden, vorzugsweise Laktose, Disacchariden und Kombinationen davon.

5. Verfahren gemäß einem der Ansprüche 1 bis 4, worin Schritt b) in einem Fließbettprozessor durchgeführt wird.

6. Verfahren nach einem der Ansprüche 1 bis 5, worin in Schritt c) ein Verdünnungsmittel, vorzugsweise Laktose, und ein Schmiermittel, vorzugsweise Magnesiumstearat, zu den Granulaten zugegeben wird.

7. Verfahren nach Anspruch 6, worin das Gewichtsverhältnis der in Schritt b) und in Schritt c) verwendeten Verdünnungsmittel zwischen 2 und 3, vorzugsweise 2,1 und 2,5 liegt.

8. Verfahren nach einem der Ansprüche 1 bis 7, weiter einschließend den Schritt des Beschichtens der in Schritt d) erhaltenen festen Form.

9. Verfahren nach einem der Ansprüche 1 bis 8, weiter einschließend, nach Schritt b) und/oder Schritt c), einen Trocknungsschritt der Granulate, bei dem die Granulate einem Gasstrom ausgesetzt oder auf eine Temperatur von 30°C bis 80°C erhitzt werden.

10. Verfahren nach einem der Ansprüche 1 bis 9, worin in Schritt b) die Verdampfung des Lösungsmittels mithilfe eines Gasstroms unterstützt wird.

11. Verfahren nach einem der Ansprüche 1 bis 10, worin die Hilfsstoffe und der aktive Inhaltsstoff vor Verwendung gesiebt und/oder die Granulate gesiebt und nach Größe geordnet werden, bevor sie in Form gebracht werden.

## Revendications

1. Procédé de préparation d'une composition pharmaceutique solide de lacidipine comprenant les étapes suivantes :
a) fourniture d'une solution de polyvinylpyrrolidone et de lacidipine dans un solvant polaire dans lequel le rapport en poids de la lacidipine à la polyvinylpyrrolidone est d'environ 10/90,
b) pulvérisation et séchage dans un lit fluide de la solution de l'étape a) sur un diluant solide, de façon à obtenir des granulés
c) éventuellement, addition aux granulés obtenus à l'étape b) d'au moins un excipient supplémentaire choisi de préférence parmi un agent glissant, un lubrifiant, un diluant et des combinaisons de ceux-ci, et
d) façonnage des granulés obtenus en une forme solide, de préférence en un comprimé.

2. Procédé selon la revendication 1, selon lequel la polyvinylpyrrolidone de l'étape a) a un poids moléculaire moyen de 10 000 à 1 000 000 g.mol⁻¹, de préférence de 20 000 à 60 000 g.mol⁻¹.

3. Procédé selon la revendication 1 ou 2, selon lequel le solvant polaire fourni à l'étape a) est choisi parmi l'éthanol et des mélanges éthanol/eau comprenant au moins 80 % (v/v) d'éthanol.

4. Procédé selon l'une quelconque des revendications 1 à 3, selon lequel le diluant de l'étape b) est choisi dans le groupe constitué de cellulose microcristalline, de monosaccharides, de préférence, de lactose, de disaccharides et de leurs combinaisons.

5. Procédé selon l'une quelconque des revendications 1 à 4, selon lequel l'étape b) est réalisée dans un processeur à lit fluidisé.

6. Procédé selon l'une quelconque des revendications 1 à 5, selon lequel à l'étape c), un diluant, de préférence le lactose, et un lubrifiant, de préférence le stéarate de magnésium, sont ajoutés aux granulés.

7. Procédé selon la revendication 6, selon lequel le rapport en poids entre le diluant utilisé à l'étape b) et le diluant ajouté à l'étape c) est compris entre 2 et 3, de préférence entre 2,1 et 2,5.

8. Procédé selon l'une quelconque des revendications 1 à 7, incluant en outre une étape d'enrobage de la forme solide obtenue à l'étape d).

9. Procédé selon l'une quelconque des revendications 1 à 8, incluant en outre, après l'étape b) et/ou l'étape c), une étape de séchage des granulés en soumettant les granulés à un courant de gaz ou en chauffant les granulés à une température de 30 °C à 80 °C.

10. Procédé selon l'une quelconque des revendications 1 à 9, selon lequel à l'étape b), l'évaporation du solvant est favorisée au moyen d'un courant de gaz.

11. Procédé selon l'une quelconque des revendications 1 à 10, selon lequel les excipients et l'ingrédient actif sont tamisés avant l'utilisation et/ou les granulés sont tamisés ou dimensionnés avant d'être mis en forme.
